# EUROPÄISCHE PATENTSCHRIFT

(11) **EP 1 911 464 B1**
(45) Veröffentlichungstag und Bekanntmachung des Hinweises auf die Patenterteilung: **16.11.2011**
(21) Anmeldenummer: 07017998.1
(22) Anmeldetag: 13.09.2007
(51) Int. Cl.: A61K 47/02, A61K 47/36, A61K 47/38, A61K 36/00

(54) **Gelartiges Basenpräparat auf Basis ätherischer Ölmischungen zur dermalen und transdermalen Anwendung**
Gel-type base compound based on essential oil mixtures for dermal and transdermal application
Préparation de base de type gel à base de mélanges d'huiles essentielles destinées à l'application dermique et transdermique

(30) Priorität: 14.09.2006 DE 102006044447
(43) Veröffentlichungstag der Anmeldung: 16.04.2008
(73) Patentinhaber: Helms, Manfred, Dr., 57392 Schmallenberg (DE)
(72) Erfinder: Helms, Manfred, Dr., 57392 Schmallenberg (DE)
(74) Vertreter: Teipel, Stephan

(56) Entgegenhaltungen:
- EP-A- 0 333 548
- WO-A-03/084501
- WO-A-2004/073676
- FR-A- 2 704 429
- JP-A- 2003 137 761

## Beschreibung

Die vorliegende Erfindung betrifft ein gelartiges Basenpräparat auf Basis ätherischer Ölmischungen zur dermalen und transdermalen Anwendung, ein Verfahren zu dessen Herstellung und die Verwendung dessen.

Ätherische Öle sind pflanzliche Öle, die je nach Herkunftspflanze bestimmte Duftstoffe enthalten. Sie unterscheiden sich von "fetten" Ölen (z.B. Sonnenblumenöl) dadurch, dass sie vollständig verdampfen, und z.B. auf Papier keine charakteristischen Flecken hinterlassen. Ätherische Öle sind Duftstoffe der Pflanzen, und ihre Aufgabe ist es, die Insekten zur Bestäubung anzulocken, Schädlinge fern zu halten, aber auch gegen Krankheiten zu schützen, die z.B. durch Bakterien oder Pilze hervorgerufen werden. Diese Wirkung macht man sich seit langem zu Nutzen, sowohl in der Kosmetikindustrie als auch in der ärztlichen Therapie. So gibt es einige nicht verschreibungspflichtige Arzneimittel auf pflanzlicher Basis, welche ihre Hauptwirkung durch ätherische Öle erhalten. So ist eine hervorragende schleimlösende Wirkung bei Katarren der oberen Atemwege, Bronchitis, etc. von den ätherischen Ölen des Eukalyptus bekannt. Den ätherischen Ölen einer Fenchel-Kümmel-Anis-Mischung wird z.B. eine Wirkung gegen Blähungen und Krämpfe im Magen-Darm-Bereich nachgesagt, bei Entzündungen im Mund- und Rachenraum sollen die ätherischen Öle von Salbei und Kamille helfen.

Bei der Aromatherapie werden ätherische Öle zur Beeinflussung von Gesundheit und Wohlbefinden angewendet. Sie ist Bestandteil der Phytotherapie (Pflanzenheilkunde) und Teil komplementärmedizinischer Methoden. Die Aromatherapie ist in Deutschland in einer berufsergänzenden Ausbildung zu erlernen. Bei der Aromatherapie wird mittels Duftstoffen auf verschiedene Weisen auf den menschlichen Körper eingewirkt. So wird einerseits der Geruchssinn angesprochen, was mit einer Sinneswahrnehmung verbunden mit verschiedenen Nebeneffekten (Gefühlseindruck, Erinnerung, reflektorische Beeinflussung verschiedener Körperfunktionen, etc.) einhergeht. Des Weiteren können insbesondere ätherische Öle eingenommen oder inhaliert werden, was eine direkte Wirkung auf den Körper hat. So ist insbesondere Lavendelöl als beruhigend, Thymian als aktivierend, Jasminöl als stark anregend bekannt, und Orangen- und Zitronenöl sollen die Stimmung aufhellen.

Als "biologische Antibiose" wird die antibiotische Eigenschaft einiger ätherischer Öle bezeichnet, wodurch sie sich gut für die Prophylaxe und zur Behandlung leichter Infekte eignen.

Zusammensetzungen zur Verwendung in der Aromatherapie, die ätherische Öle enthalten, sind im Stand der Technik bekannt. Die bekannten Zusammensetzungen besitzen jedoch eine Reihe von Nachteilen. Zum einen besteht bei einer Zusammensetzung, die wässrige Inhaltsstoffe und die fettlöslichen ätherischen Öle enthält die Gefahr der Entmischung. Auch durch Einsatz verschiedener Emulgatoren kann einer Entmischung oft nicht über lange Zeiträume entgegengewirkt werden. Ein weitaus größeres Problem besteht jedoch darin, dass ätherische Öle im Allgemeinen sehr oxidationsempfindlich sind. Eine lange Haltbarkeit und Anwendbarkeit entsprechender Zusammensetzungen ist somit nicht gewährleistet, ohne dass die Qualität dieser deutlich leidet.

Die WO 2004/073676 offenbart ein Gel, das Natriumhydroxid, Pouzzolan, Xanthan und ein ätherisches Öl enthält. Die WO 03/084501 offenbart ein Gel, das Natrium hydroxid, ein Stärkederivat und Xanthan Gum enthält.

Es besteht somit ein Bedarf an Mitteln auf Basis ätherischer Ölmischungen, die die Probleme des Standes der Technik nicht aufweisen und insbesondere eine lange Haltbarkeit und Anwendbarkeit der enthaltenen ätherischen Öle gewährleisten.

Überraschenderweise wurde gefunden, dass ein Mittel, basierend auf einem gelartigen Basenpräparat, welchem ätherische Öle zugefügt wurden, diese Aufgabe erfüllt.

Die vorliegende Erfindung betrifft daher ein gelartiges Basenpräparat zur dermalen und transdermalen Anwendung am menschlichen und tierischen Körper, gemäß Anspruch 1.

Der Erfindung liegt generell die Erkenntnis zu Grunde, dass dann, wenn ein basisches Präparat Hydroxide enthält, eine praktische Darstellung in Pulverform nicht möglich, zumindest aber äußerst problematisch ist. Da Hydroxide eine ausgeprägt hydroskopische Wirkung aufweisen, nehmen sie bereits bei der Verarbeitung Wasser aus der Luft auf, so dass es nicht, oder nur unter Verwendung weiterer Rohstoffe möglich ist, ein rieselfähiges Pulver herzustellen. Das Pulver neigt dann überdies in Folge des durch Hydroxide vermittelten hygroskopischen Effekts zur Bildung sehr fester, in Wasser nur schwer löslicher Konglomerate. Nur durch eine stabile Gelform sind diese Nachteile grundsätzlich vermeidbar.

Das erfindungsgemäße gelartige Basenpräparat enthält einen Gelbildner, bestehend aus mindestens zwei bis vorzugsweise vier Komponenten aus der Gruppe der Polymere, die vorzugsweise natürlichen Ursprungs sind. Erfindungsgemäß verwendbare Polymere sind Agar Agar, Caarageen, Johannesbrotkernmehl, Guarmehl, Stärke und deren Derivate, insbesondere Xanthan Gum, Alginsäure und Alginate, Cellulose und deren Derivate, insbesondere Carboxymethylcellulose und/oder Kieselsäure, die als anorganischer Gelbildner eingesetzt werden kann. Die Verwendung der Kieselsäure als Gelbildner ist besonders bevorzugt, da hierdurch eine Gelbildung des Basenpräparats, das einen pH-Wert über 8,5 aufweist, trotz des alkalischen pH-Werts gewährleistet werden kann. Dies geschieht insbesondere dadurch, dass die im Neutralen eher unlösliche kolloidale Kieselsäure im alkalischen Medium teilweise löslich ist und hierdurch der Aufbau von polymeren Kieselsäurestrukturen bewirkt wird. Kolloidale Kieselsäure als anorganischer Gelbildner bildet mit Wasser weißliche Pasten und wird u.a. als Thixotropier- und Vedickungsmittel z.B. in Zahnpasten (pH-Wert 6-8) eingesetzt.

Neben der Verwendung der Kieselsäure als Gelbildner spielt sie eine vorteilhafte Rolle bei der Verwendung des Basenpräparats zur dermalen und transdermalen Anwendung, da sie bei der Anwendung ebenfalls von den Zellen des menschlichen Körpers aufgenommen wird und hier von Nutzen ist. Es wird ihr unter anderem eine hilfreiche Wirkung bei der Neubildung von Haut, Haaren, Nägeln, Zähnen, Knochen und (Binde-)Gewebe, insbesondere eine Entsäuerung im Bereich des Nervengewebes nachgesagt. Des Weiteren reguliert die Kieselsäure die Schweißabsonderung und unterstützt somit die Ausscheidungsfunktion der Haut. Unter anderem ist sie bei Nierensteinen und Nierengrieß behilflich und unterstützt das Ausleiten von Eiter über die Lymphe.

Neben der bevorzugt eingesetzten Kieselsäure können als Gelbildner andere organische Polymere, insbesondere Polysaccharide, eingesetzt werden. Diese bilden strukturell unterschiedliche Gelstrukturen, die allein meist jedoch nicht ausreichen, um bei alkalischen pH-Werten und hohen Salzkonzentrationen eine ausreichende Gelbildung sicher zu stellen. Daher werden die organischen Polymere bevorzugt in Kombination mit Kieselsäure als Gelbildner eingesetzt, wobei mindestens zwei, vorzugsweise bis zu vier verschiedene Gelbildner zum Einsatz kommen.

Das erfindungsgemäße gelartige Basenpräparat weist einen alkalischen pH-Wert von 8,5 bis 12,5, bevorzugt 9,5 bis 12,5, z.B. oberhalb von 10 auf.

Des Weiteren weist das erfindungsgemäße gelartige Basenpräparat eine hohe Konzentration an gelösten oder ungelösten Salzen auf, wobei die Gesamtsalzkonzentration wenigstens 10 und bis zu 70 Gewichtsprozent, bezogen auf das Gesamtgewicht des Präparats, vorzugsweise 20 bis 60 Gewichtsprozent beträgt, so lange das erfindungsgemäße gelartige Basenpräparat keine Spurenelementsalze enthält. Enthält das erfindungsgemäße gelartige Basenpräparat Spurenelementsalze, so beträgt die Gesamtsalzkonzentration 3 bis 40 Gewichtsprozent, bezogen auf das Gesamtgewicht des Präparats, vorzugsweise von 4 bis 30 Gewichtsprozent.

Der hohe Salzgehalt bei gleichzeitig hohem pH-Wert des erfindungsgemäßen gelartigen Basenpräparats stellt hohe Anforderungen an die eingesetzten Gelbildner, da im Allgemeinen Gelstrukturen bei hohem pH-Wert und hohem Salzgehalt zerstört werden. Dies bedeutet, dass unmittelbar nach der Herstellung ein unter Umständen noch festes und stabiles Gel mit zunehmender Lagerung an Konsistenz verliert. Die ungelösten, unter Umständen fein verteilten schwerlöslichen Bestandteile, wie z.B. Mineralsalze können sich bei unzureichender Stabilität und Festigkeit des Gels absetzen. Dies führt jedoch zu einer extrem erschwerten und nicht mehr möglichen Verwendung des Produktes. Konventionelle Gelbildner können unter den genannten Bedingungen des hohen pH-Wertes neben hohen Salzkonzentrationen entweder keine genügende Gelbildung gewährleisten, oder sie müssen in unvertretbar hohen Konzentrationen eingesetzt werden. Das Einsetzten sehr hoher Konzentrationen von Gelbildnern führt jedoch neben dem inakzeptablen hohen Materialaufwand dazu, dass sich das Endprodukt oft nur noch schwer oder nicht mehr in Wasser löst, was ebenfalls nicht akzeptabel ist.

Die alleinige Verwendung nur eines der genannten Gelbildner führt aufgrund des hohen Salzgehaltes und des hohen pH-Wertes nicht zu stabilen gelartigen Zusammensetzungen. Durch die oben genannte erfindungsgemäße Kombination von mehr als zwei bis maximal vier der oben genannten Gelbildner wird eine ausreichende Gelbildung auch bei hohem pH-Wert und hohem Salzgehalt gewährleistet. Insbesondere kann ein gelartiges Basenpräparat hergestellt werden, das längere Zeit stabil ist, und insbesondere längere Zeit eine Viskosität von vorzugsweise über 10.000 mPas aufweist, z.B. über einen Zeitraum von 24 bis 36 Monaten. Hierdurch wird u. a. erreicht, dass eine Kristallisation der gelösten Salze und/oder eine Agglomeration ungelöster, suspendierter Salze verhindert wird. Das Gel bleibt in einem Temperaturbereich von 4°C bis 40°C stabil, verträgt über mehrere Stunden auch Temperaturen um ca. 80°C und ermöglicht so auf Grund der Produkthomogenität selbst bei längerer Lagerung eine gleichmäßige Dosierung durch den Verbraucher.

Die Menge der einzusetzenden Gelbildner aus der Kombination der Kieselsäure mit einem organischen Polymer oder aus zwei Polymeren aus der Liste der organischen Polymere ist so auszuwählen, dass es zu einer Ausbildung von stabilen Gelstrukturen und Viskositäten mit ausreichender Haltbarkeit kommt. Der jeweilige prozentuale Anteil der einzelnen Gelbildner sowie die Gesamtmenge kann bei der erfindungsgemäßen, oben beschriebenen Kombination im Vergleich zum Stand der Technik erheblich reduziert werden. Somit genügt ein Anteil des Gelbildners von bis zu 3 Gewichtsprozent, bezogen auf die Gesamtzusammensetzung, vorzugsweise bis zu 1 Gewichtsprozent, zur Herstellung eines erfindungsgemäßen gelartigen Basenpräparats.

Das erfindungsgemäße gelartige Basenpräparat enthält eine basische Komponente. Diese besteht vorzugsweise aus Hydroxiden und/oder Hydroxidcarbonaten und/oder Carbonaten und/oder basischen Phosphaten von mindestens einem Element der Gruppe Kalium, Natrium, Calcium und/oder Magnesium. Als Naturstoffe sind insbesondere Muschelkalk oder Dolomite als basische Komponente bevorzugt. Die basische Komponente ist in dem gelartigen Basenpräparat in einer Menge vorhanden, damit dieses einen pH-Wert von zwischen 8,5 und 12,5, vorzugsweise zwischen 9,5 und 12,5 aufweist.

Das erfindungsgemäße gelartige Basenpräparat enthält weiterhin mindestens ein ätherisches Öl. Als für die "Aromatherapie" geeignete ätherische Öle sind insbesondere Kiefernnadelöl, Öle von Fichte, Rottanne, Koriander, Oregano, Sassafras, Brustwurz, Liebstöckel, Sandelholz und Blütenessenzen von Nelke, Rose und Flieder in den erfindungsgemäßen Basenpräparaten verwendbar.

Durch die Anwesenheit von Salzen bei der Applikation der ätherischen Öle durch das erfindungsgemäße Basenpräparat mit hoher Salzkonzentration wird insbesondere die perkutane Absorption der ätherischen Öle verbessert.

Einerseits gelangen die ätherischen Öle in die oberen Hautschichten und können hier entsprechend ihrer Zusammensetzung verschiedene Wirkungen hervorrufen, z.B. desinfizierende Wirkung, wie sie dem Lavendelöl und seinen verdünnten Lösungen, aber auch vielen anderen ätherischen Ölen nachgesagt wird. Andererseits gelangen die ätherischen Öle in geringer Konzentration auch durch Inhalation in die Lunge und haben hier ebenfalls z.B. desinfizierende Wirkung, werden aber auch in geringen Konzentrationen mit der Atemluft ins Blut aufgenommen und können auf diese Weise Signale im zentralen Nervensystem aktivieren, z.B. beruhigend, anregend oder stimulierend wirken. Die besondere Wirkung von ätherischen Ölen auf die Psyche liegt an dem intensiven Zusammenspiel von Riechzellen, Riechnerv, Riechhirn und dem limbischen System, das die entsprechenden Reaktionen hervorruft.

Es hat sich gezeigt, dass in dem durch die erfindungsgemäßen gelartigen Basenpräparate zur Verfügung gestellten hochbasischen Milieu, insbesondere von pH-Werten oberhalb von 10, die ansonsten sehr oxidationsempfindlichen ätherischen Öle gut geschützt werden können und damit eine lange Haltbarkeit und Anwendbarkeit von mindestens 24 bis 36 Monaten der Zusammensetzung gewährleistet ist, ohne dass die Qualität, insbesondere die der ätherischen Öle, darunter leidet.

Der hohe Salzgehalt der erfindungsgemäßen Basenpräparate gewährleistet weiterhin einen Schutz gegen mikrobiellen Befall, da diese Salzkonzentration in Kombination mit dem hohen pH-Wert für die meisten Bakterien, Viren und Pilze als Lebensraum ungeeignet ist.

In einer bevorzugten Ausführungsform enthält das erfindungsgemäße gelartige Basenpräparat insbesondere zur besseren Einarbeitung und zur Verhinderung der Entmischung der ätherischen Öle einen Emulgator. Als solche Emulgatoren können sowohl auch in Lebensmitteln verwendbare natürliche Emulgatoren, wie z.B. Lecithin, als auch technisch hergestellte, wie z.B. PEG-35 Castor Oil verwendet werden. Besonders bevorzugt ist der Einsatz eines Alkohol-Wasser-Gemisches als Emulgator für die ätherischen Öle. Dieses hat neben der Wirkung als Emulgator noch den Effekt, dass sich das ätherische Öl in ihm gut mischt und so eine ausgezeichnete Verteilung des ätherischen Öls im salzhaltigen Gel gewährleistet. Daneben können andere Bestandteile des erfindungsgemäßen gelartigen Basenpräparats als Emulgator verwendet werden. So ist insbesondere bei Alginaten eine Emulgatorwirkung bekannt. Somit genügt bei einer erfindungsgemäßen Zusammensetzung für ein gelartiges Basenpräparat, das Alginsäure oder ein Derivat derer umfasst, die Emulgatorwirkung dieser, sodass kein weiterer Emulgator zugesetzt werden muss. Vorteilhaft an einer solchen Herstellung ist die Tatsache, dass es in diesem Fall nicht erforderlich ist Konservierungsmittel einzusetzen, weil der hohe Salzgehalt sowohl der Mineralstoff- als auch der Spurenelementgele, einschließlich des hohen pH-Wertes oberhalb pH = 10 im Konzentrat einen mikrobiellen Befall verhindert.

Neben den oben genannten für die Aromatherapie bevorzugten ätherischen Ölen könnten auch ein oder mehrere Öle ausgewählt aus der Gruppe Limettöl dest., Orangenöl, Eucalyptusöl, Fichtennadelöl, Bergamottöl, Citronenöl, Cedernholzöl, Myrrhenöl, Geraniumöl afrik., Sandelholzöl, Latschenkiefernöl, Abs. Jasminöl, Muskateller Salbeiöl, Ylangöl, Lavendelöl, Patchouliöl, Rosmarinöl, Vetiveröl, Citronenöl, Minzöl, Orangeöl, Grapefruitöl, Basilikumöl, Rosenholzöl, Lemongrasöl, Sanddornöl, Litseaöl, Buccoblätteröl, Amyrisöl, Calmusöl, Fenchelöl, Muskatöl, Neroliöl, Sternanisöl und Wacholderöl verwendet werden.

In einer Ausführungsform enthält das erfindungsgemäße gelartige Basenpräparat Mineralstoffsalze. Bevorzugte Mineralstoffsalze sind die Karbonate und/oder Phosphate und/oder Hydroxide und/oder Hydroxidcarbonate wenigstens einem der Elemente der Gruppe Calcium, Kalium, Magnesium, und Natrium. Für den Fall dass das erfindungsgemäße gelartige Basenpräparat lediglich Mineralstoffsalze, jedoch keine Spurenelementsalze enthält, weist es eine hohe Salzkonzentration von wenigstens 10 Gewichtsprozent bis zu 70 Gewichtsprozent, insbesondere 20 bis 60 Gewichtsprozent, bezogen auf das Gesamtgewicht des Präparates auf.

In einer Ausführungsform enthält das erfindungsgemäße gelartige Basenpräparat weiterhin mindestens ein Spurenelement. Die Spurenelemente werden im Allgemeinen in Form verschiedener Salze der Elemente einer ersten Gruppe bestehend aus Eisen, Zink, Kupfer, Mangan, Chrom, Jod, Fluor, Selen und Molybdän und/oder einer zweiten Gruppe bestehend aus Brom, Lithium, Bor, Kobalt, Germanium, Nickel, Vanadium, Rubidium und Zinn eingesetzt. Die eingesetzten Spurenelementsalze sind wasserlöslich, was zu Lösungen führt, oder wasserunlöslich, was zu Suspensionen führt. Das Spurenelementsalze enthaltende gelartige Basenpräparat besitzt bedingt durch die Gelbildner-Konzentration ein breites Spektrum an einsetzbaren Salzkonzentrationen. So beträgt der Anteil an gelösten oder ungelösten Salzen vorzugsweise wenigstens 3 Gewichtsprozent bis 40 Gewichtsprozent, insbesondere 4 Gewichtsprozent bis 30 Gewichtsprozent, bezogen auf das Gesamtgewicht der Zusammensetzung.

Zur Stabilisierung der Oxidationsstufe der Spurenelemente in den erfindungsgemäßen gelartigen Basenpräparaten wird vorzugsweise mindestens eine organische Säure, vorzugsweise ausgewählt aus der Gruppe bestehend aus Ameisensäure, Essigsäure, Zitronensäure, Ascorbinsäure, Benzoesäure, Weinsäure, Glucuronsäure, Zimtsäure, Milchsäure, Salizylsäure, Oxalsäure, Malonsäure, Bernsteinsäure, Glutarsäure, Adipinsäure und/oder Äpfelsäure eingesetzt. Besonders bevorzugt wird als organische Säure Zitronensäure und Ascorbinsäure eingesetzt. Die organische Säure bildet mit dem Spurenelement einen Komplex, wodurch die Oxidationsstufe des Spurenelements stabilisiert wird.

Die erfindungsgemäßen gelartigen Basenpräparate können des Weiteren wasserlösliche und/oder fettlösliche Vitamine enthalten. Als Vitamine können vorzugsweise Vitamin A, B1, B2, B6, B12, C, E, Folsäure, Biotin, Pantothensäure und/oder Niacin eingesetzt werden. Bei der Verwendung von Vitamin A und E ist die Verwendung in Kombination mit einem der oben genannten Emulgatoren, um eine schonende Einarbeitung in das erfindungsgemäße Basenpräparat zu ermöglichen, bevorzugt. Die erfindungsgemäßen gelartigen Basenpräparate eignen sich besonders auf Grund ihres basischen Charakters zur gleichzeitigen Supplementierung der Vitamine neben der Anwendung der ätherischen Öle, da zum Einen die wasserlöslichen Vitamine im Basischen bekanntermaßen stabiler sind als im Sauren, zum Anderen aber auch fettlösliche Vitamine durch die Kombination mit fettlöslichen ätherischen Ölen vorteilhaft über die Haut resorbiert werden. Auf diese Weise erhält man ein sehr vielseitiges und wirksames Spurenelement-Präparat, welches nahezu den gesamten Bereich der Spurenelemente beinhaltet und somit in der Lage ist, als Nahrungsergänzungsmittel aber auch als Arzneimittel einem breiten Spektrum an Mangelerscheinungen entgegenzuwirken.

Bevorzugte Anwendungsgebiete des erfindungsgemäßen gelartigen Basenpräparats liegen in dermalen oder transdermalen Anwendungen, wie im Kosmetikbereich, z.B. als gelförmiger Bestandteil der wässrigen Phase von kosmetischen und pharmazeutischen Emulsionen, wie Salben, Cremes, Lotionen usw., oder in der Aromatherapie, z.B. als Badezusatz.

Besonders vorteilhaft lässt sich das erfindungsgemäße gelartige Basenpräparat als Badezusatz, z.B. eines basischen Ausleitungsbades verwenden, im Rahmen einer inneren und äußeren Aromatherapieanwendung. Hierdurch werden durch die Kombination der Inhalation der ätherischen Öle mit deren desinfizierenden Eigenschaften besonders gute Effekte erzielt. Dies wird insbesondere durch die gleichzeitige Einwirkung der Wärme in Kombination mit Ruhe im Rahmen des Entspannungsbades verstärkt.

Die erfindungsgemäßen gelartigen Basenpräparate können im Rahmen einer Aromatherapie zur Entsäuerung und Entgiftung des Körpers über die Haut zur Vermeidung von hiermit verbundenen Krankheitszuständen sowie für einen weit gefächerten dermalen und transdermalen Bereich angewendet werden, insbesondere bei trockener Haut, Psoriasis, Neurodermitis, Windeldermatitis, Soor- und Pilzerkrankungen, chronische, schlecht verheilende Wunden (z.B. Ulcus cruris), diabetische Ulcera und bei Erkrankungen des rheumatischen Formenkreises, z.B. als äußerliche Rheuma- und Gelenksalbe und als unterstützende Maßnahme zu einer inneren Therapie.

In einer Ausführungsform werden die erfindungsgemäßen gelartigen Basenpräparate, die Mineralstoffsalze und diejenigen, die Spurenelementsalze enthalten, jeweils allein oder in Kombination miteinander verwendet, wobei eine abwechselnde Anwendung im Abstand von etwa zwei Tagen für Vollbäder und im täglichen Abstand bei Teilbädern, Auflagen und Wickeln besonders vorteilhaft ist.

Zur Erhöhung des Verbrauchernutzens stellen die erfindungsgemäßen gelartigen Basenpräparate ein Konzentrat dar, d.h. sie müssen vor Anwendung entsprechend verdünnt werden, z.B. in Wasser. Der Verbraucher rührt beispielsweise 25 - 50 g des erfindungsgemäßen gelartigen Basenpräparats in ein Voll- bzw. Teilbad ein und erhält so die Anwendungskonzentration.

Ein im Stand der Technik bestehendes erhebliches Problem ist die Herstellung eines alkalischen Gemisches, das Spurenelemente enthält, da viele Spurenelemente komplexe Redoxreaktionen in verschiedenen pH-Bereichen, insbesondere im Alkalischen, eingehen können. So kommt es zur Oxidation, wie z.B. beim Eisen, oder es bilden sich schwerlösliche oder unlösliche Hydroxide oder Carbonate, z.B. der Elemente Eisen, Zink, Kupfer, Mangan, Molybdän usw.

Erfindungsgemäß wurde gefunden, dass durch eine spezielle Reihenfolge bei der Herstellung des erfindungsgemäßen gelartigen Basenpräparats sowohl im Bezug auf die Reihenfolge der Zugabe der Inhaltsstoffe als auch im Bezug auf den entsprechenden vorliegenden pH-Wert bei der Zugabe dieses Problem gelöst werden kann und insbesondere unerwünschte Redoxreaktionen, die zur Umwandlung der Spurenelementverbindungen in unwirksame oder nicht mehr vom Körper aufnehmbare Verbindungen führt, vermieden werden kann.

Die vorliegende Erfindung betrifft somit ebenfalls ein Verfahren zur Herstellung eines erfindungsgemäßen gelartigen Basenpräparats. Das erfindungsgemäße Verfahren besteht darin, eine wässrige Lösung der Substanzen zur Stabilisierung der Oxidationsstufen der Spurenelemente vorzulegen, die vorzugsweise einen pH-Wert unter 7 aufweist, in der ausgewählte Spurenelemente, insbesondere solche, die im pH-Wert unter 7 keine unerwünschten Redoxreaktionen eingehen, ggf. unter Erwärmen einzumischen. Dies sind z.B. Eisen, Zink, Mangan, Chrom, Molybdän, Cobalt, Nickel und Vanadium. Anschließend wird die basische Komponente bis zum Erreichen des gewünschten alkalischen pH-Wertes der Zusammensetzung zugegeben, gefolgt von den Spurenelementen, die im Alkalischen keine unerwünschten Redoxreaktionen eingehen. Das sind z.B. Kupfer, Fluor, Jod, Selen, Brom, Lithium, Bor, Germanium, Rubidium und Zinn. Sollen die erfindungsgemäßen gelartigen Basenpräparate statt Spurenelementen Mineralstoffverbindungen enthalten, werden diese zuerst in der Gesamtmenge an Wasser vorgelegt und diese Mischung erwärmt. In die erwärmte Mischung wird ebenfalls die basische Komponente bis zum Erreichen des gewünschten alkalischen pH-Wertes der Zusammensetzung zugegeben. In die jeweils erwärmten Mischungen wird schließlich der trocken eingewogene Gelbildner bzw. das Gemisch der Gelbildner zugegeben und intensiv homogenisiert. Nach leichter Abkühlung des Gels erfolgt die Zugabe der Mischung aus den ätherischen Ölen und der ggf. eingesetzten Emulgatoren. Nach ausreichender Abkühlung können ggf. noch Vitamine hinzu gegeben werden. Die nach dem Abkühlen erhaltene viskose Flüssigkeit kann anschließend abgefüllt werden.

Durch das erfindungsgemäße beschriebene Verfahren wird ein gelartiges Basenpräparat zur Verfügung gestellt, dessen stabile Gelstruktur in Abhängigkeit von Salzgehalt und Gelbildnerzusammensetzung zwischen 24 bis 36 Monaten lagerstabil bleibt. Bevorzugt können bei dem erfindungsgemäßen Verfahren als Stabilisator der Oxidationsstufe der Spurenelemente oben angegebene organische Säuren eingesetzt werden.

Die unter anderem nach dem erfindungsgemäßen Verfahren erhaltenen erfindungsgemäßen gelartigen Basenpräparate besitzen insbesondere durch die Verwendung der oben genannten Gelbildnerkombination eine gute Löslichkeit in Wasser, was für ein entsprechend markttaugliches Produkt erforderlich ist. Der Verbraucher kann beispielsweise zur Herstellung eines Vollbades das erfindungsgemäße gelartige Basenpräparat ohne Probleme in Wasser auflösen bzw. suspendieren. Hinzu kommt, dass in dem hochbasischen Milieu der erfindungsgemäßen gelartigen Basenpräparate die ansonsten sehr oxidationsempfindlichen ätherischen Öle sehr gut geschützt werden und damit eine lange Haltbarkeit und Anwendbarkeit von mindestens 24 bis 36 Monaten gewährleistet ist, ohne dass die Qualität, insbesondere die der ätherischen Öle, darunter leidet.

Die ausgezeichnete dermale und transdermale Wirkung der erfindungsgemäßen gelartigen Basenpräparate lässt sich einerseits über den basischen pH-Wert und die hohe Salzkonzentration erklären, was zu einer Anregung der Diffusion und der Osmose und damit zur Verbesserung der Ausleitung von Säuren, Schlacken und Giftstoffen über die Haut führt. Andererseits gelangen die Mineralstoffe und Spurenelemente über die Haut auch in den Körper, hervorgerufen durch den Konzentrationsgradienten der einzelnen Ionen zwischen dem erfindungsgemäßen Basenpräparat bzw. der entsprechenden Anwendungsform, wie dem Badewasser, und dem Körper. Daneben entfalten die ätherischen Öle gleichzeitig ihre Wirkung, die durch die Anregung der Diffusion und der Osmose noch verstärkt wird.

Die Erfindung betrifft weiterhin die Verwendung eines Gelbildners, der mindestens zwei Substanzen aus der Gruppe der organischen Polymere wie oben angegeben enthält, zur Herstellung eines erfindungsgemäßen gelartigen Basenpräparats auf Basis ätherischer Öle, was eine Salzkonzentration von 10 bis 70 Gewichtsprozent, wenn es Mineralstoffsalze und keine Spurenelementsalze enthält, und 3 bis 40 Gewichtsprozent, wenn es Spurenelementsalze enthält, bezogen auf die Zusammensetzung, und einen pH-Wert von 8,5 bis 12,5 aufweist.

Schließlich betrifft die vorliegende Erfindung die Verwendung des erfindungsgemäßen gelartigen Basenpräparats in der Aromatherapie, insbesondere als Badezusatz.

Die Zusammensetzung des erfindungsgemäßen gelartigen Basenpräparats und das Verfahren zu dessen Herstellung wird nachstehend an einigen Beispielen beschrieben.

### Beispiel 1)

Herstellung des erfindungsgemäßen gelartigen Basenpräparates auf Basis ätherischer Öle enthaltend Mineralstoffsalze.

In der Gesamtmenge an Wasser werden die Mineralstoffsalze gelöst/suspendiert und die Mischung erwärmt. Anschließend wir die basische Komponente bis zu dem Erreichen eines pH-Wertes von 9 bis 11 zugegeben. Unter intensivem Mischen wurden die trocken eingewogenen und vorher vorgemischten Gelbildner zugegeben und die Mischung auf eine Temperatur von 70 bis 90°C bei Homogenisierungsgeschwindigkeiten von 2.500 bis 3.500 U/min vermischt. Nach dem Abkühlen der Mischung auf ca. 40°C bis 50°C erfolgt bei ebenfalls hohen Homogenisierungsgeschwindigkeiten die Zugabe der Mischung aus ätherischen Ölen, Alkohol und Emulgatoren. Nach weiterer Abkühlung der Lösung auf ca. 40°C wurden die entsprechenden Vitaminpräparate zugegeben. Es wurde eine viskose Flüssigkeit erhalten, die so in Flaschen abgefüllt eine stabile Gelstruktur aufwies. In Abhängigkeit von Salzzusammensetzung und Gelbildnerzusammensetzung blieb das Gel zwischen 24 und 36 Monate lagerstabil.

### Beispiel 2)

Herstellung des erfindungsgemäßen gelartigen Basenpräparats auf Basis ätherischer Öle enthaltend Spurenelementsalze

Nach Herstellung einer wässrigen Lösung der organischen Säuren zur Stabilisierung der Oxidationsstufe der Spurenelemente in der Gesamtmenge an verwendetem Wasser wurden einige Spurenelementsalze zugegeben und anschließend erwärmt. Im Anschluss wurden in die Spurenelementmischung so viel an basischer Komponente hinzu gegeben, bis ein pH-Wert von 9 bis 11 erreicht wurde. Einige Spurenelementsalze wurden erst jetzt zugegeben. Unter intensivem Mischen wurden die trocken eingewogenen und vorgemischten Gelbildner zugegeben und die Mischung auf eine Temperatur von 70 bis 90°C bei Homogenisierungsgeschwindigkeiten von 2.500 bis 3.500 U/min vermischt. Nach Abkühlung des Gels auf ca. 40°C bis 50°C erfolgte ebenfalls bei hohen Homogenisatorgeschwindigkeiten die Zugabe der Mischung aus ätherischen Ölen, Alkohol und Emulgatoren. Nach der Abkühlung der Lösung auf ca. 40°C wurden die entsprechenden Vitaminpräparate zugegeben. Es wurde eine viskose Flüssigkeit erhalten, die so in Flaschen abgefüllt eine stabile Gelstruktur aufwies. In Abhängigkeit von Salzzusammensetzung und Gelbildnerzusammensetzung blieb das Gel zwischen 24 und 36 Monate lagerstabil.

### Beispiel 3)

Beispielhafte Zusammensetzung dreier erfindungsgemäßer gelartiger Basenpräparate auf Basis ätherischer Öle beinhaltend Mineralstoffsalze und dreier erfindungsgemäßer gelartiger Basenpräparate auf Basis ätherischer Öle enthaltend Spurenelementsalze

### Basische Aromatherapie-Gele auf Basis von Mineralstoffen

| A) | | |
|---|---|---|
| **Nr.** | **Handelsbezeichnung des Bestandteils** | **Gewichtsprozent** |
| 1 | VE-Wasser | 27,00 - 85,10 |
| 2 | Mineralstoffsalze | 70,00 - 10,00 |
| 3 | Organische und/oder anorganische Gelbildner | 3,00 - 0,40 |
| 4 | Vitamine | 3,00 - 0,50 |
| 5 | Emulgatoren | 7,00 - 3,00 |
| 6 | Alkohol | 5,00 - 1,00 |
| 7 | Ätherische Öle | 5,00 - 0,50 |

| B) | | |
|---|---|---|
| **Nr.** | **Handelsbezeichnung des Bestandteils** | **Gewichtsprozent** |
| 1 | VE-Wasser | 32,50 |
| 2 | Mineralstoffsalze | 50,00 |
| 3 | Organische und/oder anorganische Gelbildner | 1,50 |
| 4 | Vitamine | 3,00 |
| 5 | Emulgatoren | 5,50 |
| 6 | Alkohol | 4,50 |
| 7 | Ätherische Öle | 3,00 |

| C) | | |
|---|---|---|
| **Nr.** | **Handelsbezeichnung des Bestandteils** | **Gewichtsprozent** |
| 1 | VE-Wasser | 72,65 |
| 2 | Mineralstoffsalze | 20,00 |
| 3 | Organische und/oder anorganische Gelbildner | 0,75 |
| 4 | Vitamine | 1,00 |
| 5 | Emulgatoren | 3,00 |
| 6 | Alkohol | 1,50 |
| 7 | Ätherische Öle | 1,10 |

### Basische Aromatherapie-Gele auf Basis von Spurenelementen

| A) | | |
|---|---|---|
| **Nr.** | **Handelsbezeichnung des Bestandteils** | **Gewichtsprozent** |
| 1 | VE-Wasser | 30,00 - 92,10 |
| 2 | Organische Säuren | 19,00 - 1,25 |
| 3 | Spurenelementsalze | 11,00 - 0,60 |
| 4 | Basische Komponenten | 20,00 - 1,15 |
| 5 | Organische und/oder anorganische Gelbildner | 3,00 - 0,40 |
| 6 | Vitamine | 3,00 - 0,50 |
| 7 | Emulgatoren | 7,00 - 3,00 |
| 8 | Alkohol | 5,00 - 1,00 |
| 9 | Ätherische Öle | 5,00 - 0,50 |

| B) | | |
|---|---|---|
| **Nr.** | **Handelsbezeichnung des Bestandteils** | **Gewichtsprozent** |
| 1 | VE-Wasser | 43,65 |
| 2 | Organische Säuren | 15,00 |
| 3 | Spurenelementsalze | 8,50 |
| 4 | Basische Komponenten | 16,50 |
| 5 | Organische und/oder anorganische Gelbildner | 2,60 |
| 6 | Vitamine | 2,50 |
| 7 | Emulgatoren | 5,00 |
| 8 | Alkohol | 3,50 |
| 9 | Ätherische Öle | 2,75 |

## Patentansprüche

1. Gelartiges Basenpräparat zur dermalen und transdermalen Anwendung am menschlichen und tierischen Körper, das eine basische Komponente, bis zu drei Gewichtsprozent eines Gelbildners, der mindestens zwei Substanzen aus der Gruppe der Polymere ausgewählt aus Agar Agar, Carrageen, Johannesbrotkernmehl, Guarmehl, Stärke und Stärkederivate, Cellulose und Cellulosederivate, Alginsäure und Alginate sowie Xanthan Gum und/oder Kieselsäure umfasst, und mindestens ein ätherisches Öl umfasst, **dadurch gekennzeichnet, dass** es weiterhin Mineralstoffsalze bei einer Gesamtsalzkonzentration des Basenpräparates von 10 bis 70 Gewichtsprozent, oder Spurenelementsalze, bei einer Gesamtsalzkonzentration des Basenpräparats von 3 bis 40 Gewichtsprozent enthält, und dass der pH-Wert des Basenpräparats von 8,5 bis 12,5 beträgt.

2. Gelartiges Basenpräparat gemäß Anspruch 1, **dadurch gekennzeichnet, dass** die basische Komponente wenigstens ein Hydroxid und/oder Hydroxidcarbonat und/oder Carbonat und/oder basisches Phosphat wenigstens eines Elementes aus der Gruppe Kalium, Natrium, Calcium und/oder Magnesium umfasst.

3. Gelartiges Basenpräparat gemäß Anspruch 1, das 20 bis 60 Gewichtsprozent Mineralstoffsalze enthält.

4. Gelartiges Basenpräparat gemäß Anspruch 1, **dadurch gekennzeichnet, dass** das Spurenelementsalz ausgewählt ist aus einem oder mehreren Salzen der Gruppe der Elemente bestehend aus Eisen, Zink, Kupfer, Mangan, Chrom, Jod, Fluor, Selen und Molybdän und/oder der Gruppe der Elemente bestehend aus Brom, Lithium, Bor, Kobalt, Germanium, Nickel, Vanadium, Rubidium und Zinn.

5. Gelartiges Basenpräparat gemäß Ansprüchen 1 oder 4, das, wenn es Spurenelemente enthält, weiterhin eine organische Säure enthält.

6. Gelartiges Basenpräparat gemäß einem der vorstehenden Ansprüche, **dadurch gekennzeichnet, dass** es weiterhin einen Emulgator enthält.

7. Gelartiges Basenpräparat gemäß vorstehendem Anspruch, **dadurch gekennzeichnet, dass** es als Emulgator Ethanol enthält.

8. Gelartiges Basenpräparat gemäß einem der vorigen Ansprüche, **dadurch gekennzeichnet, dass** es weiterhin mindestens ein Vitamin enthält.

9. Verfahren zur Herstellung eines gelartigen Basenpräparats gemäß Anspruch 1, **dadurch gekennzeichnet, dass** eine Mischung einer organischen Säure zum Stabilisieren der Oxidationsstufe von Spurenelementsalzen mit einem oder mehreren Spurenelementsalzen oder eine wässrige Lösung/Suspension von Mineralstoffsalzen vorgelegt wird, anschließend erwärmt wird, vor, während oder nach dem Erwärmen der pH-Wert der Lösung durch Zugabe einer basischen Komponente erhöht wird, ggf. weitere Spurenelementsalze zugegeben werden und anschließend ein Gemisch von Gelbildnern, wie in Anspruch 1 beschrieben, zugegeben wird, nach dem Abkühlen ein ätherisches Öl, ggf. gemischt mit einem Emulgator zugegeben wird, und nach weiterem Abkühlen ggf. Vitamine zugegeben werden.

10. Verfahren gemäß Anspruch 9, worin das ätherische Öl gemischt mit einem Emulgator eingesetzt wird.

11. Verfahren gemäß einem der Ansprüche 9 oder 10, wobei die basische Komponente ausgewählt ist aus der Gruppe der Hydroxide und/oder Hydroxidcarbonate und/oder Carbonate und/oder basischen Phosphate der Elemente Kalium, Natrium, Calcium und/oder Magnesium.

12. Verwendung eines Gelbildners, der mindestens zwei Substanzen aus der Gruppe der Polymere ausgewählt aus Agar Agar, Carrageen, Johannesbrotkernmehl, Guarmehl, Stärke und Stärkederivate, Cellulose und Cellulosederivate, Alginsäure und Alginate sowie Xanthan Gum und/oder Kieselsäure enthält, zur Herstellung eines gelartigen Basenpräparats gemäß Anspruch 1.

13. Verwendung eines gelartigen Basenpräparats gemäß Ansprüchen 1 bis 8 in der Aromatherapie.

14. Verwendung eines gelartigen Basenpräparats gemäß Ansprüchen 1 bis 8 als Badezusatz.

## Claims

1. Gel-like base preparation for the dermal and transdermal administration to the human and animal body, comprising a basic component, up to 3 % by weight of a gelling agent containing at least two substances selected from the polymer group of agar agar, carrageen, carob gum, guar gum, starch and starch derivatives, cellulose and cellulose derivatives, alginic acid and alginates as well as xanthan gum and/or silica, and at least one ethereal oil, **characterized in that** it further comprises mineral salts, at a total salt concentration of the base preparation of 10 to 70 % by weight, or trace element salts, at a total salt concentration of the base preparation of 3 to 40 % by weight, and **in that** the pH value of the base preparation is from 8.5 to 12.5.

2. Gel-like base preparation according to claim 1, **characterized in that** the basic component comprises at least one hydroxide and/or hydroxy carbonate and/or carbonate and/or basic phosphate of at least one element selected from the group of potassium, sodium, calcium and/or magnesium.

3. Gel-like base preparation according to claim 1 comprising 20 to 60 % by weight of mineral salts.

4. Gel-like base preparation according to claim 1, **characterized in that** the trace element salt is selected from one or more salts of the group of elements consisting of iron, zinc, copper, mangan, chromium, iodine, fluorine, selenium and molybdenum and/or the group of elements consisting of bromine, lithium, boron, cobalt, germanium, nickel, vanadium, rubidium and tin.

5. Gel-like base preparation according to claim 1 or 4 which, in case it contains trace elements, further contains an organic acid.

6. Gel-like base preparation according any one of the preceding claims, **characterized in that** it further contains an emulsifier.

7. Gel-like base preparation according to the preceding claim, **characterized in that** the emulsifier contained is ethanol.

8. Gel-like base preparation according to any one of the preceding claims, **characterized in that** it further contains at least one vitamin.

9. Process for the preparation of a gel-like base preparation according to claim 1, **characterized in that** a mixture of an organic acid with one or more trace element salts or an aqueous solution/suspension of mineral salts for stabilizing the oxidation state of the trace element salts is prepared and subsequently heated, the pH value of the solution is increased by addition of a basic component prior to, during or after the heating, optionally further trace element salts are added and subsequently a mixture of gelling agents, as described in claim 1, is admixed, an ethereal oil, optionally mixed with an emulsifying agent, is added after cooling and after further cooling vitamins are optionally added.

10. Process according to claim 9, wherein the ethereal oil is used in mixture with an emulsifier.

11. Process according to any of claims 9 or 10, wherein the basic component is selected from the group of hydroxides and/or hydroxy carbonates and/or carbonates and/or basic phosphates of the elements potassium, sodium, calcium and/or magnesium.

12. Use of a gelling agent comprising at least two of the substances selected from the polymer group of agar agar, carrageen, carob gum, guar gum, starch and starch derivatives, cellulose and cellulose derivatives, alginic acid and alginates as well as xanthan gum and/or silica for the preparation of a gel-like base preparation according to claim 1.

13. Use of a gel-like base preparation according to claims 1 to 8 in aroma therapy.

14. Use of a gel-like base preparation according to claims 1 to 8 as bath additive.

## Revendications

1. Préparation de base de type gel pour l'application dermique et transdermique sur le corps humain et animal, qui comporte une composante basique, jusqu'à trois pour cent en poids d'un gélifiant qui comporte au moins deux substances du groupe des polymères choisis parmi l'agar-agar, le carragène, la farine de graines de caroube, la farine de guar, l'amidon et les dérivés d'amidon, la cellulose et les dérivés de cellulose, l'acide alginique et les alginates ainsi que la gomme xanthane et/ou l'acide silicilique, et comporte au moins une huile éthérée, **caractérisée en ce qu'**elle contient en outre des sels minéraux à une concentration totale de la préparation de base de 10 à 70 pour cent en poids, ou des sels d'oligo-éléments à une concentration totale de la préparation de base de 3 à 40 pour cent en poids, et que la valeur du pH de la préparation de base est de 8,5 à 12,5.

2. Préparation de base de type-gel selon la revendication 1 **caractérisée en ce que** la composante basique comprend au moins un hydroxyde et/ou un hydroxycarbonate et/ou un carbonate et/ou un phosphate basique d'au moins un élément du groupe du potassium, sodium, calcium et/ou magnésium.

3. Préparation de base de type gel selon la revendication 1 qui contient de 20 à 60 pour cent en poids de sels minéraux.

4. Préparation de base de type gel selon la revendication 1 **caractérisée en ce que** le sel d'oligo-élément est choisi parmi un ou plusieurs sels du groupe des éléments composés par le fer, le zinc, le cuivre, le manganèse, le chrome, l'iode, le fluor, le sélénium et le molybdène, et/ou du groupe des éléments composés par le brome, le lithium, le bore, le cobalt, le germanium, le nickel, le vanadium, le rubidium et l'étain.

5. Préparation de base de type gel selon les revendications 1 ou 4 qui, lorsqu'elle contient des oligo-éléments, contient en outre un acide organique.

6. Préparation de base de type gel selon l'une des revendications précédentes **caractérisée en ce qu'**elle contient en outre un émulsifiant.

7. Préparation de base de type gel selon la revendication précédente **caractérisée en ce qu'**elle contient de l'éthanol en tant qu'émulsifiant.

8. Préparation de base de type gel selon l'une des revendications précédentes **caractérisée en ce qu'**elle contient en outre au moins une vitamine.

9. Procédé pour la fabrication d'une préparation de base de type gel selon la revendication 1 **caractérisé en ce qu'**on présente un mélange d'un acide organique pour la stabilisation de l'étape oxydative des sels d'oligo-éléments avec un ou plusieurs sels d'oligo-éléments ou une solution/suspension aqueuse de sels minéraux, qu'on le réchauffe ensuite, qu'on augmente la valeur du pH de la solution par addition d'une composante basique avant, pendant ou après le réchauffage, éventuellement, des oligo-éléments supplémentaires peuvent être ajoutés et finalement, un mélange de gélifiants, tels que décrits en revendication 1 est ajouté, après le refroidissement, une huile éthérée, éventuellement mélangée avec un émulsifiant, est ajoutée, et après un autre refroidissement, des vitamines sont éventuellement ajoutées.

10. Procédé selon la revendication 9 dans laquelle on emploie de l'huile éthérée mélangée à un émulsifiant.

11. Procédé selon l'une des revendications 9 ou 10 où la composante basique est choisie dans le groupe des hydroxydes et/ou des hydroxycarbonates et/ou des carbonates et/ou de phosphate basique des éléments potassium, sodium, calcium et/ou magnésium.

12. Utilisation d'un gélifiant qui contient au moins deux substances du groupe des polymères choisis parmi l'agar-agar, le carragène, la farine de graines de caroube, la farine de guar, l'amidon et des dérivés d'amidon, la cellulose et des dérivés de cellulose, l'acide alginique et des alginates ainsi que la gomme xanthane et/ou l'acide silicilique pour la fabrication d'une préparation de base de type gel selon la revendication 1.

13. Utilisation en aromathérapie d'une préparation de base de type gel selon les revendications 1 à 8.

14. Utilisation d'une préparation de base de type gel selon les revendications 1 à 8 en tant qu'additif pour le bain.
